# EUROPEAN PATENT SPECIFICATION

(11) **EP 2 773 953 B1**
(45) Date of publication and mention of the grant of the patent: **15.05.2019**
(21) Application number: 12845004.6
(22) Date of filing: 02.11.2012
(51) Int. Cl.: G01N 33/18, G01N 31/22, C02F 1/00, G06G 1/08, C02F 1/66, C02F 1/76, C02F 103/42

(54) **COMPARATOR FOR USE IN IMPROVING WATER QUALITY**
KOMPARATOR ZUR VERWENDUNG BEI DER VERBESSERUNG EINER WASSERQUALITÄT
COMPARATEUR DESTINÉ À ÊTRE UTILISÉ POUR AMÉLIORER LA QUALITÉ DE L'EAU

(30) Priority: 04.11.2011 AU 2011904608; 10.11.2011 US 201161557929 P; 28.02.2012 AU 2012900772; 29.02.2012 US 201261604677 P
(43) Date of publication of application: 10.09.2014
(73) Proprietor: Zodiac Group Australia Pty Limited, New South Wales 2164 (AU)
(72) Inventor: MASTIO, Emmanuel, Smithfield, New South Wales 2164 (AU)
(74) Representative: Ipside
(86) International application number: PCT/AU2012/001351
(87) International publication number: WO 2013/063654

(56) References cited:
- CN-U- 201 437 521
- DE-A1- 2 626 013
- DE-A1- 3 314 234
- DE-U1- 20 316 987
- FR-A1- 2 199 601
- US-A1- 2004 144 699

## Description

### TECHNICAL FIELD

A comparator for use in improving the water quality of a body of water is disclosed. A test strip may be used with the comparator, and may be applied in testing and improving the water quality of a swimming pool, however, the disclosure is to be broadly interpreted, in that the comparator may also be applied in testing and improving the water quality of a pond, aquarium, spa, hot tub, or other body of water.

### BACKGROUND ART

The treatment of water in backyard pools, hot tubs, spas, aquariums and the like, is required to ensure that various qualities and parameters, such as chemical, physical and/or biological characteristics, are within given acceptable ranges. When a given chemical, physical and/or biological characteristic falls outside these acceptable ranges, it is necessary for the water to be treated to prevent someone or something becoming unwell from exposure or characteristics being imbalanced, etc. The water can be treated in a number of ways, including chemical dosing, irradiation, filtration, etc.

To determine whether various chemical, physical and/or biological characteristics are within the acceptable ranges, it is necessary to perform tests on the water. The type of test required generally depends on the characteristic being tested. Testing, interpretation and correlation of the results can be quite complicated, and it is often necessary to have a professional analyse the results to determine the best procedure for treatment of the water to improve its quality.

One manner of testing water quality utilises so-called test strips. A test strip is placed in the water and removed after a pre-determined time. One or more sections of the test strip may be adapted to react to different characteristics of the water. For example, one part of the test strip may test for pH and another part for total hardness. That part of the test strip will then change colour, depending on, e.g., the pH of the water, etc. The colour on the test strip is then compared with a colour on a reference chart to determine the current pH of the water. It is then necessary to determine what the 'ideal' pH should be, and the type of corrective action necessary to alter the pH of the water.

US 6,413,473 discloses a reference table which can be used to determine a certain mass of a chemical to be added to pool to alter the water chemistry thereof. After testing the current chemical conditions of the pool, the test strip is compared and correlated with the reference table and the required mass of a corrective chemical is referred to on the reference table. It is then necessary to weigh the chemical to ensure the water is being treated with the correct dosage.

It is also known from DE20316987U1 to use a comparator with movable portions to evaluate a test strip.

DE2626013A1 and FR2199601 A1 disclose mechanical devices that indicate a suitable water treatment based on numerical input parameters.

There are various electronic systems which use electronic sensors to detect various water quality parameters. Some systems show the result of the detected parameter on an electronic display, such as in US 2004/0144699. Other systems are more sophisticated and automatically dispense the required volume of chemical, dependent on the detected parameters, such as in CN 201364483 or CN201437521.

The above references to the background art do not constitute an admission that the art forms a part of the common general knowledge of a person of ordinary skill in the art. The above references are also not intended to limit the application of the comparator as disclosed herein.

### SUMMARY OF THE DISCLOSURE

According to a first aspect, there is disclosed a comparator for use in improving the water quality of a body of water. The comparator is adapted for comparing one or more results, whereby the or each result corresponds to a measured parameter indicative of water quality. The comparator is further adapted to indicate a quantitative dosage of a water treating agent required to improve the quality of the water, responsive to one or more of the measured parameter(s). The comparator comprises at least one movable portion which, when moved to the result of the parameter measured, indicates the quantitative dosage of water treating agent to be used to treat the body of water to improve the quality thereof.

The comparator can provide a simplified approach to the treatment of bodies of water, without the expense and complication of electronic systems. Use of a movable portion allows the indicated dosage of a water treating agent to be more prominently displayed than the non-indicated dosages. For example, the non-relevant dosages may be obscured from view, with only the indicated dosage being revealed, or a line or arrow may be used to 'point' or draw attention to the indicated dosage. The 'indicated' dosage may also refer to a recordation of the required dosage. In this regard, the movable portion may be provided with stops, ridges, grooves, or the like, such that once it has been moved through to the correct result, the stops prevent the movable portion from being accidently moved, as additional force is required to move the movable portion past the stops. This effectively records the result and thus the quantitative dosage required.

The movable portion may take the form of a sleeve. The sleeve may have an opening or window therein to allow an indicated dosage to be viewed once the sleeve has been moved. In this regard, the various potential dosages may be obscured from view until the sleeve is moved, which movement can be according to the result of the measured parameter. For example, when the sleeve is moved, only the indicated dosage may be revealed. This avoids confusion of the indicated dosage and ensures that the correct dosage is indicated and thus dispensed into the body of water to improve the quality thereof. It also allows the result on a test strip, for example, to be compared with the comparator and the quantitative dosage determined before the test strip overreacts (thus, again, preventing an incorrect dosage from being dispensed). This allows the correct dosage to be determined, without actually having to dispense the dosage at the same time (i.e. a number of results can be compared before each water treating agent is added to the body of water). This ensures the correct result is 'recorded' or 'frozen' on the comparator, even though the test strip may continue to react beyond its optimal time for comparing the result with the comparator.

Alternatively, the movable portion or sleeve may comprise an indicating line or arrow, which can point or draw attention to the quantitative dosage indicated.

Once the parameter has been measured, a result indicative of the water quality parameter being measured is obtained. The result is colourimetric. The comparator may provide an array or range of potential results against which the result of the measured parameter can be compared. The comparative result on the comparator that is closest to the measured result, i.e. a colour, may be adapted to indicate the quantitative dosage of water treating agent required to improve the water quality. In this regard, the movable portion of the comparator can be moved to the result of the parameter measured to indicate the quantitative dosage of water treating agent required. It should be noted that where only one parameter indicative of water quality is measured and appropriate corrective action taken, the water quality may only be improved in respect of that parameter measured. Other parameters or characteristics of water quality may not have been improved, thus the overall quality of the water may still be poor. In any case "improving the quality of a body of water" is to be interpreted to include improving just one parameter.

The parameter indicative of water quality may include, but is not limited to: acidity; alkalinity; total alkalinity; biguanide; bromine; free bromine; chloride; chlorine; free chlorine; free and combined chlorine; conductivity; copper; cyanuric acid; hardness; calcium hardness; total hardness; hydrogen peroxide; iron; manganese; mineral/salt; monopersulfate; NaCl (salt); nitrate; oxidation reduction potential (ORP); ozone; pH; phosphate; quaternary ammonium compounds (QAC); etc.

The water treating agent may include, but is not limited to: sodium bicarbonate; sodium carbonate; sodium bisulphate; sodium hypochlorite; chlorine; hydroxyethylidene diphosphonic acid complex; calcium chloride; hydrochloric acid; trichloroisocyanuric acid; sodium tetraborate pentahydrate; cyanuric acid; copper sulphate pentahydrate; boric acid; sodium dichloroisocyanurate dihydrate; calcium hypochlorite; potassium peroxymonopersulphate; aluminium sulphate; natural clarifier; cationic polyectrolyte; aluminium chlorhydrate; aluminium oxide; poly[oxyethylene(dimethyliminio)ethylene-(dimethyliminio)ethylene dichloride]; cupric ammonium complex; benzalkonium chloride; copper - triethanolamine complex (as copper); quaternary ammonium chloride; isocyanuric acid; sodium metabisulphite; 1-hydroxyethylidene-1,1-diphophonic acid; citric acid monohydrate; oxalic acid; magnesium sulphate heptahydrate; aluminosilicate; etc.

The quantitative dosage may be indicated in a number of ways, including the provision of units for measurement. For example, the quantitative dosage may be weight, volume, or some other form of discrete measurement (such as a bag, bottle, sachet, tablet, etc).

In one embodiment, the comparator may be located on a vessel. The vessel may be for holding an item that would generally be associated with the testing of water, such as water treating agents, test strips, sampling and specimen bottles, chemical test kits, etc.

In one embodiment, the comparator may further comprise one or more test strips. Each test strip may be used to obtain a result which is indicative water quality of the parameter being tested. In this regard, the parameter being measured may show a colourimetric response to the test strip. The colour obtained on the test strip can then be compared to the comparator, the latter of which shows representative results of the parameter being measured. The colour on the comparator which most closely represents the colour shown on the test strip then indicates the quantitative dosage required to improve the parameter of the water quality being measured. Alternatively, a small sample of the water may be collected from the body of water and a colourimetric indicator may be added thereto. The indicator causes the water sample to change colour, dependent on the result of the parameter being measured. The coloured water sample may then be compared with the comparator.

In one embodiment, the or each test strip may be able to test for one or more of the parameters indicative of water quality at a respective indicator region. This can simplify and reduce the effort required to maintain the body of water at an acceptable water quality as a single test can provide insight into a number of parameters that may need rectification. A portion of the test strip adjacent to the or each indicator region may comprise a transparent region. This may facilitate the comparison of the measured result with the potential results provided on the comparator.

In one embodiment, the vessel may further comprise a test strip holder located on a visible portion of the vessel. This holder may be arranged to allow the test strip to be placed in the vicinity of the comparator, to simplify comparison of the measured parameter with the comparator. The test strip may be positionable at or in the holder after it has been dipped in the water to be tested. Alternatively, an unused test strip may be positioned at or in the holder on the vessel, and the vessel dipped in the water.

In one embodiment, once the test strip is positioned at or in the test strip holder, the comparator can be adapted to indicate the quantitative dosage. In one embodiment, the test strip holder may be movable. A movable test strip holder may be useful where multiple parameters are being measured, allowing the test strip to be moved adjacent to the corresponding comparator. In one embodiment, the test strip holder can be separated on the vessel from where the quantitative dosage is indicated. Separating the test strip holder from where the quantitative dosage is indicated encourages a user to wait until the measured parameter has completed measurement, thus preventing comparison before completion and ensuring the correct quantitative dosage is indicated.

In one embodiment, the test strip holder may be positioned such that the test strip, when held therein, overlies the indicated results / moveable portion. This allows a test strip, of the kind disclosed in the fifth aspect (below), to be positioned in front of the moveable portion of the comparator. The transparent region of the test strip allows the indicated results to be readily seen therethrough, as the moveable portion is moved, to allow a comparison between alternative results. It also prevents potential contamination of the test strip if it were to be placed behind the moveable portion(s) of the comparator. At test strip which comprises no transparent region may also be used, however, it may be preferable to have each indicated result as a band that is wider than the test strip to allow easy comparison. In an alternative embodiment, the test strip holder may be positioned such that the or each movable portion overlies the test strip, when the test strip is positioned at or in the holder. The depth of the holder may be such that the indicator regions on the test strip do not contact the back of the movable portion, thus also preventing contamination of the test strip.

A lower portion of the test strip holder may be shaped so as to receive thereat a similarly shaped end of a test strip. This may prevent incorrect insertion of a test strip, or prevent an unsuitable test strip from being inserted (i.e. one that does not correspond to the parameters of the comparator). Thus, the test strip holder and test strip can co-operate to ensure correct insertion for use.

In one embodiment the vessel on which the comparator is located may comprise a holder for unused (fresh) test strips for measuring the one or more parameters. This enables an unused test strip to be removed from the vessel and then used to test the parameter, and then compared to the comparator that is located on the same vessel. This streamlines comparison, as the vessel for holding fresh test strips is already at hand.

In an alternative embodiment, the vessel may contain one or more of the water treating agents. This allows the result indicative of the water quality to be compared with the comparator located on the vessel and the water treating agent to be thereafter rapidly dosed into the water. Further, if the vessel contains a plurality of water treating agents, each water treating agent may be contained in a separate compartment within the vessel. If each water treating agent is located in the one vessel, again they can be rapidly dispensed into the water.

The comparator may further comprise at least one second moveable portion that alters the indicated quantitative dosage to account for one or more factors which affect the quantitative dosage. The one or more factors may include: the volume of the body of water; water temperature; frequency of use; number of users of the body of water; or seasonal factors, such as summer, winter, spring, etc. This allows a "single comparator system" to be produced which can be used by a number of users, having pools, spas, aquariums, etc, of different volumes, different water temperatures, different usage requirements, etc, whilst ensuring that the correct dosage of a water treating agent is being indicated.

In one embodiment, the second moveable portion can additionally be locked against movement. This may be useful for a household user where the volume, for example, of a pool spa, aquarium, etc, does not substantially alter. This allows the volume of the pool, spa, aquarium, etc to be initially set on the comparator, preventing the incorrect dosage due to volume being indicated.

In one embodiment, the comparator may further comprise a dosage device for dispensing the indicated quantitative dosage required to improve the quality of water. This can simplify dosage of the water with the water treating agent and ensure accurate dosages are being dispensed, without the need to accurately measure or weigh the water treating agent. The dosage device may be a scoop, sachet, bag, pellet, tablet, bottle, container or bucket, etc.

The comparator may further comprise a timing device. The timing device may display an optimal time for comparing the result of the parameter measured with the comparator (e.g. an optimal elapsed time after a test strip has been dipped in the body of water). This assists with ensuring that the result of the measured parameter is not compared with the comparator before sufficient time has been allowed for the result to generate, thus preventing the incorrect dosage being indicated. Further, the timing device also prevents the result of the measured parameter being compared with the comparator significantly after the optimal time which may also affect the measured parameter result obtained, again preventing the incorrect dosage being indicated.

According to a second aspect, there is disclosed a system for improving the water quality of a body of water in which a parameter indicative of the water quality is measured. The system comprises a comparator against which a result of the measured parameter is compared.

The comparator of the system of the second aspect is as defined in the first aspect.

According to a third aspect, there is disclosed a method of improving the water quality of a body of water in which a parameter indicative of the water quality is measured. The result of the parameter measured is compared with a comparator.

The comparator of the method of the third aspect is as defined in the first or second aspects.

According to a fourth aspect, there is disclosed a comparator for use in improving the water quality of a body of water. The comparator may be adapted for comparing one or more results, whereby the or each result corresponds to a measured parameter indicative of water quality. The comparator may comprise at least one movable portion which, when moved to the result of the parameter measured, indicates a particular result. In this regard, the result shown on the comparator can be 'recorded' or 'frozen' once the movable portion indicates the particular result over other non-selected results. This prevents an incorrect result from a test strip, for example, which has overreacted being compared with the comparator.

In one embodiment, when the movable portion is moved to the particular result of the parameter measured, the comparator indicates a quantitative dosage of water treating agent required to improve the quality of the water. The quantitative dosage may be indicated on a separate portion of the comparator to where the result of the measured parameter is located. This allows results to be determined on one part of the comparator and the other part of the comparator can be used to determine the quantitative dosage. The results and dosages may, for example, be located on opposite sides of a comparator, such as on opposite sides of a vessel. The quantitative dosage may be determined from an array of representative results with the relevant dosages, or the movable portion(s) may, for example, comprise an indicative line or arrow, a window, or other mechanism to draw attention to, or indicate, the quantitative dosage required.

The comparator of the fourth aspect may be otherwise as defined in the first, second or third aspects.

There is disclosed a test strip comprising at least one indicator region adapted to indicate a parameter related to water quality. A portion of the test strip adjacent to the or each indicator region comprises a transparent region. In this context, the term "adjacent" is intended to include both where portions of the regions are in contact with each other, and where portions of the regions are situated near to, or close to, each other but are not in contact. When such a test strip is used in conjunction with the comparator, system and method disclosed in the first to fourth aspects, the transparent region of the test strip may overlie the moveable portion, thus obtaining an unobstructed view of comparative results for the or each parameter measured by the test strip. In this regard, the test strip may have more than one indicator region, with each region adapted to indicate a different parameter related to water quality.

This arrangement, whereby the test strip can be positioned over the comparator, means that each indicator region does not become "contaminated" by e.g. contact with the comparator, or a test strip holder, etc.

The transparent region may comprise an aperture, or hole, to provide an unobstructed view of the comparative results. Alternatively, the transparent region may comprise a transparent material. In this case, the transparent material may be detachable from the remainder of the test strip, thereby forming an aperture or hole. For example, the transparent material may be frangibly connected to the surrounding test strip. This allows the transparent material, should it change colour or become opaque, to be detached from the remainder of the test strip so that the results on the test strip can still be readily compared with the comparative results on the movable portion of the comparator.

The test strip may be adapted to indicate the direction the test strip should be used. This enables a simple reference to ensure that the parameters measured on the test strip are being compared with the correct comparative results. For example, the test strip may indicate the correct direction by a specific shaping of an end, or by indicia or markings on the test strip. In this regard, an end of the test strip may taper to or be provided with a point, or the test strip may have an arrow thereon. A tapered/pointed end may be used to distinguish different types of tests strips, for example, from other test strips that test for the same parameters indicative of water quality. The different test strips may alternatively have ends of different shape that, when used with the incorrect comparator, may cause misalignment of the transparent regions and the comparative results, thus preventing use. In a further alternative, each test strip that tests for a given parameter may have one specific end shape, indicia or markings, and each test strip that tests for another given parameter may have a different end shape, indicia or markings, and so on.

The test strip disclosed may be used with a comparator as disclosed in the first or fourth aspects. Similarly, the test strips disclosed may be used with the system as disclosed in the second aspect, or in the method disclosed in the third aspect.

Where the test strip has a shaped end, and the comparator has a test strip holder with a correspondingly shaped recess, and when a test strip having an end of a different shape is attempted to be inserted into the test strip holder, insertion will be prevented and/or the indicator region(s) will be misaligned with the comparative results on the moveable portions of the comparator. Similarly, test strips which indicate a range of different parameters or test strips with a different sequence of parameters may have a different shaped end, which then prevents the results being compared with an incorrect comparator. For example, where the test strip comprises one or more transparent regions adjacent to the or each indicator region, the transparent regions may be misaligned with the comparative results on the moveable portions of the comparator, whereby the comparative results are unable to be seen and read (i.e. obscured) to prevent comparison of the indicator region on the test strip with the comparative result on the comparator.

### BRIEF DESCRIPTION OF THE DRAWINGS

Notwithstanding any other forms which may fall within the scope of the comparator, system and method as set forth in the Summary, specific embodiments will now be described, by way of example only, with reference to the accompanying drawings in which:
Figures 1A to 1C show a first embodiment of a comparator and dosage system;
Figures 2A to 2C show a second embodiment of a comparator and dosage system;
Figure 3 shows a third embodiment of a comparator and dosage system;
Figures 4A and 4B show a fourth embodiment of a comparator and dosage system;
Figures 5A to 5C show a fifth embodiment of a comparator and dosage system;
Figures 6A to 6D show four examples of a test strip;
Figures 7A and 7B show a test strip being utilised with an embodiment of a comparator;
Figures 8A and 8B show schematic views of a test strip holder; and
Figures 8C and 8D show schematic views of the test strip holder shown in Figure 8A with a test strip inserted therein.

### DETAILED DESCRIPTION OF SPECIFIC EMBODIMENTS

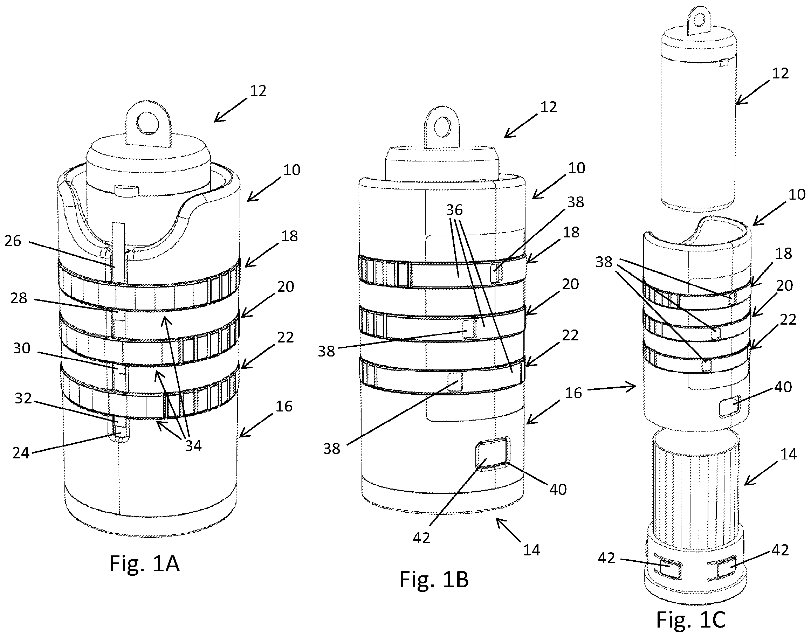

Referring firstly to Figures 1A to 1C, a first embodiment of a comparator and dosage system for use in determining the appropriate remedying action for improving the water quality of a body of water, in the form of an unused test strip bottle holder 10, is shown. Holder 10 is shown housing a test strip bottle 12 that contains unused test strips. The holder 10 is further shown having two portions, an inner sleeve 14 and an outer sleeve 16. The outer sleeve 16 further comprises three collars 18, 20, 22 that are independently movable with respect to a remainder of the outer sleeve.

The outer sleeve 16 further comprises a test strip holder slot 24 that allows the test strip to be placed (e.g. slid) therein once testing has been performed. Alternatively, the test strip may be placed into the holder slot 24 before testing takes place, and the entire holder 10 may be placed into the water for testing. The test strip 26 is shown having three testing regions 28, 30, 32. When the test strip 26 is located in the test strip holder slot 24, the three testing regions 28, 30, 32 are located in proximity to the three collars 18, 20, 22. This allows for the results of the parameters tested to be readily and reliably compared with the representative comparative results shown on the collars 18, 20, 22. For example, region 28 on test strip 26 may test for free chlorine using colourimetric comparisons. When the test strip is placed in the water, test region 28 changes colour dependent on the amount of free chlorine in the water. Potential indicative colourimetric results of the free chlorine test are shown on collar 18. Collar 18 is moved so that the colour shown on test region 28 is aligned with the colour on collar 18 which is the closest match thereto.

In this regard, collars 18, 20 and 22 each have a first portion 34 thereof providing potential indicative colourimetric results and a second portion 36 that is adapted to indicate the quantitative dosage of water treating agent required. In this embodiment, the second portions 36 of the collars are each provided with a window 38 that reveals the dosage of water treating agent which is provided on the inner sleeve 14. The inner sleeve 14 comprises an array of dosages for each of the parameters tested, and for various volumes of pools, spas, aquariums, etc. In this regard, outer sleeve 16 is movable with respect to (i.e. rotatable on) the inner sleeve 14 to adjust the dosage indicated to account for different volumes of water to be treated, with the dosage then being viewable through window 38. Once the test strip has been dipped in the water, timing is essential to ensure that the correct result on the test strip is compared with the comparative results on the comparator. This is because the testing regions on the test strip may overreact if they are left too long, thus providing a 'false' result. As the comparator has movable sleeves, the comparative result, and thus the indicated dosage, is essentially 'recorded' or 'frozen' once the movable portion has been moved. This eliminates guess work and memory issues regarding which water treating agent, in what dosage, needs to be dispensed. Rather it is possible to determine and hold the results for a number of parameters before dispensing any water treating agents.

The outer sleeve 16 is also lockable against accidental movement, to prevent the dosage being indicated for an incorrect volume. Outer sleeve 16 comprises a volume window 40. Inner sleeve 14 comprises a number of tabs 42, each of which indicate a different volume of water. When a tab 42 is positioned in window 40, the dosages indicated in windows 38 correspond to the dosages required for the volume of water shown in window 40. Tabs 42 are each part of a leaf-spring finger 44, so that each tab can "snap" into window 40 to thereby hinder rotation of sleeve 16. To alter the volume of water being indicated, when tab 42 is positioned in window 40, the tab is depressed, causing leaf-spring 44 to flex inwardly into a hollow part of sleeve 14, thus allowing the outer sleeve 16 to be rotated on the inner sleeve. This can be repeated until the correct volume of water is shown in window 40.

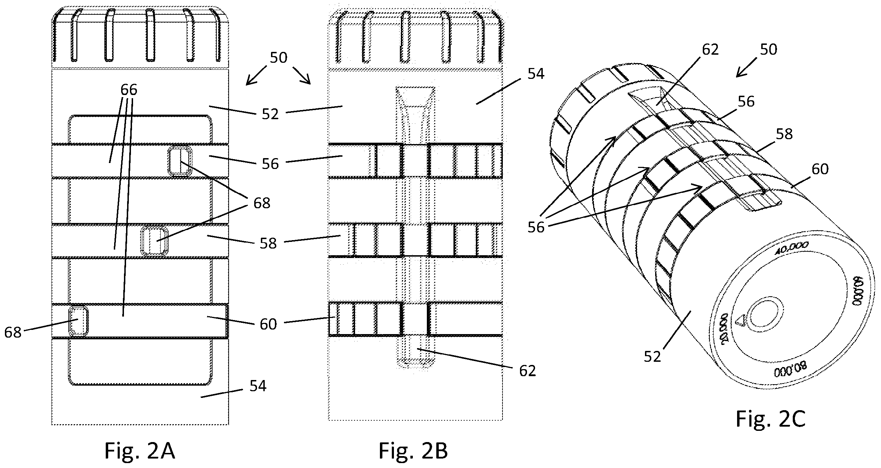

A second embodiment of a comparator 50 and dosage system is shown in Figures 2A to 2C. Comparator 50 is similar to the comparator 10 described with reference to Figures 1A to 1C, however, has been modified and simplified to provide indicated dosages for one water volume only. Comparator 50 is shown as a test strip bottle 52 that contains unused test strips and indicates dosages for water volumes of 40,000L. The bottle 52 is further shown having only one outer portion 54 that comprises three collars 56, 58, 60 that are independently movable with respect to a remainder of the outer portion.

The outer portion 54 further comprises a test strip holder slot 62 that allows the test strip to be placed (e.g. slid) therein once testing has been performed. When a test strip having three testing regions is located in the test strip holder slot 62, the three testing regions are located in proximity to the three collars 56, 58, 60. This allows for the results of the parameters tested to be readily and reliably compared with the representative comparative results shown on the collars 56, 58, 60.

In this regard, collars 56, 58, 60 each have a first portion 64 thereof providing potential indicative colourimetric results and a second portion 66 that indicates the quantitative dosage of water treating agent required. In this embodiment, the second portions 66 of the collars are again each provided with a window 68 that reveals the dosage of water treating agent. The window 68 reveals the indicated dosage for a given result. An array of dosages for each of the parameters tested is located on the outer portion 54 underneath collars 56, 58, 60. Movement of each collar 56, 58, 60 such that the colour shown on the test region of the test strip is aligned with the colour on the collar 56, 58, 60 which is the closest match thereto, positions the window 68 over the dosage indicated for each result.

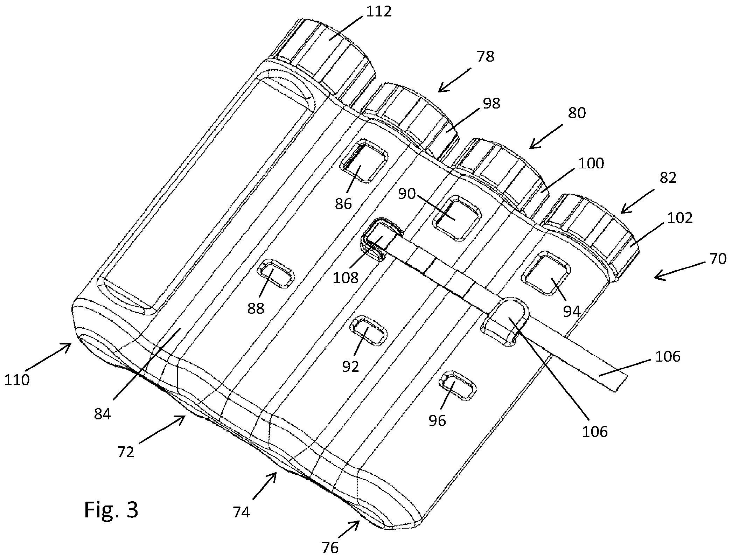

Figure 3 shows a third embodiment of a comparator 70 and dosage system to indicate dosages for water volumes of 80,000L. Comparator 70, like comparators 10 and 50, is shown having three comparative regions 72, 74, 76. Each region provides the indicative/comparative results and an indicative dosage, dependent upon the result obtained. Each comparative region 72, 74, 76 comprises a cylinder 78, 80, 82. The cylinders are located within a housing 84, which comprises two windows 86, 88; 90, 92; 94, 96 for each respective region. The first window 86, 90, 94 for each comparative region reveals a possible indicative/comparative result (e.g. shade of colour). The cylinders 78, 80, 82 can be rotated by turning the knob 98, 100, 102 to reveal the various comparative results. The second window 88, 92, 96 for each comparative region reveals a dosage for treating the water. As each cylinder 78, 80, 82 is rotated so the comparative result (e.g. shade of colour) shown in the window 86, 90, 94 matches the result of the measured parameter on an adjacent test strip, the dosage shown in the window 88, 92, 96 also rotates and is altered according to the result of the parameter measured. Alternatively, or additionally, the cylinders 78, 80, 82 may be slid in the housing 84 to reveal different quantitative dosages for different water temperatures, for example.

In this regard, comparator 70 can be provided with a projection, in the form of a tongue or tab 104, for holding a test strip 106 in the vicinity of windows 86, 90, 94. This allows the results obtained on the test strip to be easily and reliably compared with the comparative results. To enhance retention of test strip 106, a recess or slot 108 is provided for the test strip to be located therein.

Comparator 70 additionally has a fourth hollow region 110, secured with a cap 112. The hollow region may be used to store, for example, unused test strips.

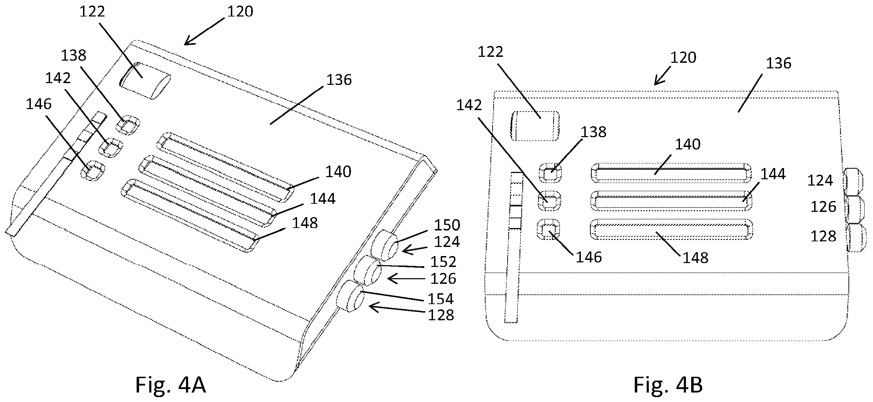

Figures 4A and 4B show a fourth embodiment of a comparator 120 and dosage system, that is similar to comparator 70 shown in Figure 3. However, in comparator 120 the indicated dosage can be altered dependent on the volume of the body of water, by rotating cylinder 122. Comparator 120 is shown having three comparative regions 124, 126, 128. Each region provides a possible indicative/comparative result (e.g. shade of colour) and an indicative dosage, dependent upon the result obtained (e.g. on a test strip). Each comparative region 124, 126, 128 comprises a rotatable cylinder 130, 132, 134. The cylinders are located within a housing 136, which comprises two windows 138, 140; 142, 144; 146, 148 for each respective region. The first window 138, 142, 146 for each comparative region reveals the indicative/comparative result (e.g. given shade of colour). Each cylinder 130, 132, 134 can be rotated by turning a respective knob 150, 152, 154 to reveal the various comparative results. The second window 140, 144, 148 for each comparative region reveals a dosage for treating the water. As each cylinder 130, 132, 134 is rotated so the comparative result shown in the window 138, 142, 146 matches the result of the measured parameter on an adjacent test strip, the array of dosages shown in the windows 140, 144, 148 also rotate and each is altered according to the result of the parameter measured. Volume cylinder 122, which is attached to a gearing system (not shown) which is also connected to a portion of cylinders 130, 132, 134, can be rotated so that the volume of water to be treated is visible, thus altering the indicated dosage shown in second window 140, 144, 148.

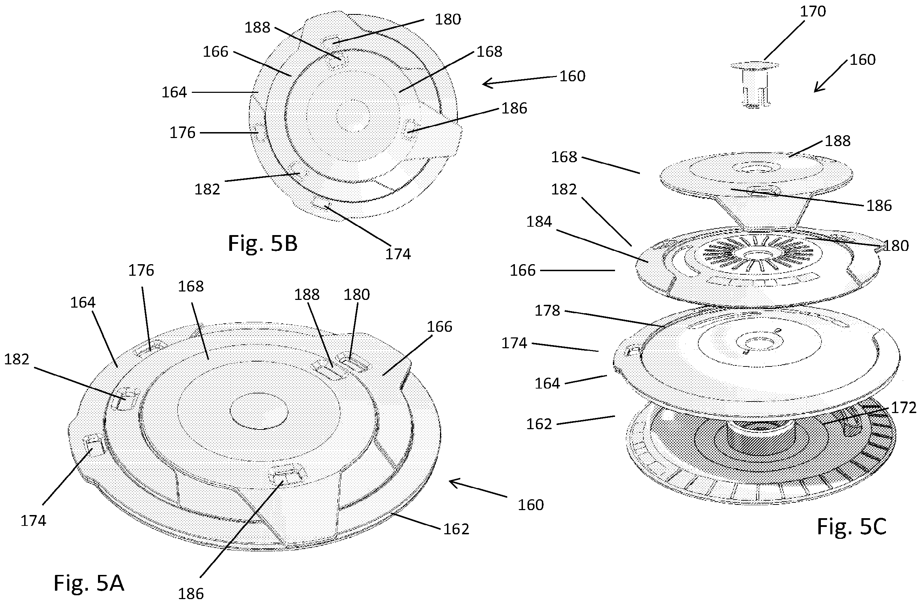

Figures 5A to 5C show a fifth embodiment of a comparator 160 and dosage system. Comparator 160 is shown having four superimposed discs 162, 164, 166, 168 held together by a centrally located press fit clip 170, although fewer or additional discs may be provided depending on the number of parameters being tested. The bottom disc 162 is overlaid by a first intermediate disc 164. Bottom disc 162 has a raised portion forming an elongate slot 172 which receives a protrusion (not shown) extending from the underside of intermediate disc 164. This limits the range of motion of intermediate disc 164. Intermediate disc 164 comprises two windows 174, 176 and its own elongate slot 178. Window 174 reveals a comparative result which is located on bottom disc 162 and, as the protrusion of intermediate disc 164 is slid along slot 172 and the intermediate disc rotates, a range of comparative results are revealed, against which the result obtained on an adjacent test strip can be compared. As intermediate disc 164 is rotated, window 176 also rotates. Window 176 reveals a dosage for treating the water and, as window 176 is moved due to the result obtained, the indicated dosage is also altered.

Second intermediate disc 166 also comprises a protrusion (not shown) extending from the underside thereof, two windows 180, 182 and its own elongate slot 184, similar to those provided in first intermediate disc 164. The second intermediate disc 166 is used to determine the dosage required to improve the water quality of a second parameter being measured in a similar manner to that described for first intermediate disc 164. Top disc 168 also comprises a protrusion (not shown) extending from the underside thereof and two windows 186, 188 and is used to determine the dosage required to improve the water quality of a third parameter being measured, in a similar manner to that described for intermediate disc 164, 166. The discs may be formed from a variety of materials, including polymers, cardboard, timber, laminate, metals, etc.

The various comparators and dosage systems described above with reference to Figures 1 to 5 can be seen to provide a range of "smart" vessels or bottles or test equipment. In this regard, the comparators and dosage systems can provide for more reliable and reproducible dosage of water bodies to remedy various parameter imbalances, and can reduce human error, false judgment, assumptions, miscalculations, etc.

The dosage result can also correspond to a dosage format. For example, a result of "2" can correspond to two scoops, two sachets, two bottles, two containers, two tablets, etc. The required format can be supplied together with the comparator, for example, in an associated container, or in the vessel itself (i.e. on which the comparator is located). The vessel may, for example, be adapted to hold e.g. unused test strips and up to three different water treating agents in the required dosage formats. These may be held in separate compartments or smaller containers within the vessel, or may be individually wrapped and labeled/coloured tablets, sachets, etc.

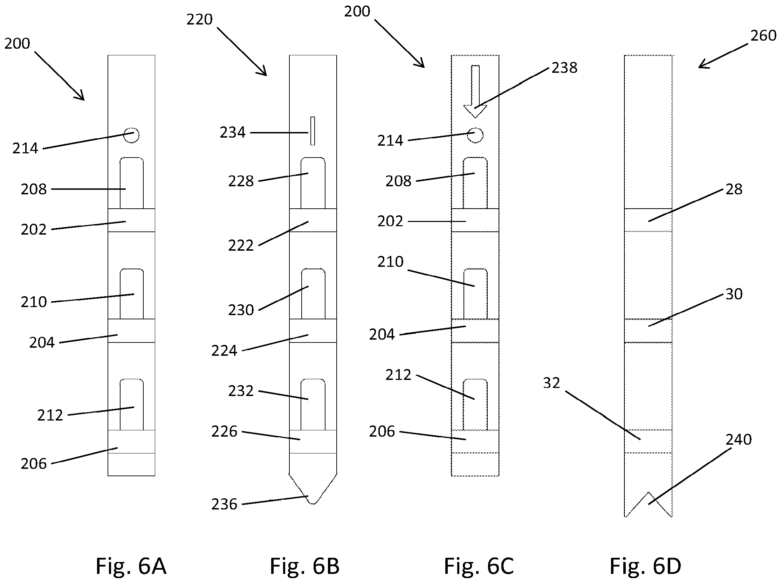

Figure 6A shows a test strip 200. Test strip 200 is similar to test strip 26, in that it also has three indicator regions, in the form of testing regions 202, 204, 206. However, located adjacent to each testing region is a transparent region, shown in this embodiment in the form of an aperture or window 208, 210, 212. Test strip 200 is also shown having a further aperture 214, which may be used to engage with (e.g. receive therein) a projection on the comparator, to ensure correct alignment of the test strip therewith. In this example, aperture 214 is circular, and would therefore engage with a similarly shaped projection (e.g. a round pin).

Figure 6B shows a second example of a test strip 220. Test strip 220 is also shown having three indicator regions, in the form of testing regions 222, 224, 226. Each testing region is shown having an adjacent transparent region, in the form of a transparent material 228, 230, 232. Test strip 220 is also shown having an aperture 234, in the shape of a rectangle. The rectangular aperture 234 is adapted to engage with a correspondingly shaped projection on a comparator. Again, this may be used to ensure that the test strip is correctly aligned with the comparator, and that the correct test strip is also being used with the correct comparator. In the embodiment of Figure 6B test strip 220 is also shown having a tapered end 236. The tapered end 236 may have a dual function, in that it indicates the direction in which the test strip should be compared with (e.g. inserted in relation to) the comparator, and can also ensure the test strip is being used with the correct comparator. For example, the tapered end indicates the direction in which the test strip should be placed into the test strip holder on the comparator. Further, a lower portion of the test strip holder (e.g. a rebate or recess) on a comparator, such as those shown in Figures 1 to 4, may be shaped to correspond to the tapered end 236 of the test strip 220. Thus, when a test strip that has a correspondingly shaped end is correctly placed into the test strip holder, the transparent regions 228, 230, 232 may overlie the comparative result regions, i.e. the moveable portions, on the comparator. However, where the tapered end 236 of the test strip 220 does not correspond to the shape in the test strip holder, the testing regions cannot be correctly aligned on the comparator, which alerts the user that either the incorrect insertion direction, comparator or test strip is being used.

Figure 6C shows a third example of a test strip 200, which is otherwise as shown in Figure 6A. However, in this example, the test strip 200 is shown having an indicia, in the form of an arrow 238, which indicates the direction in which the test strip 200 should be used (e.g. the direction that the test strip is to be compared against the comparator, or is to be inserted into the test strip holder).

Whilst the examples shown in Figures 6A to 6C depict test strips having a portion of the respective transparent regions in contact with their respective indicator regions, other embodiments may provide for the regions to be situated near, or close, to each other without being in contact.

Figure 6D shows a fourth example of a test strip 260, which is otherwise the same as test strip 26 shown in Figure 1A. However, test strip 260 is shown having a cut away portion 240 which may correspond to a similarly shaped lower region (e.g. indent, ridge, shelf, rebate or recess) of a test strip holder on a comparator. Test strip 260, unlike test strips 200 and 220, does not have a transparent region, and is thus more suitable for use with a comparator such as those shown in Figures 1 and 2, in which the test strip is placed behind (instead of over) the movable collars.

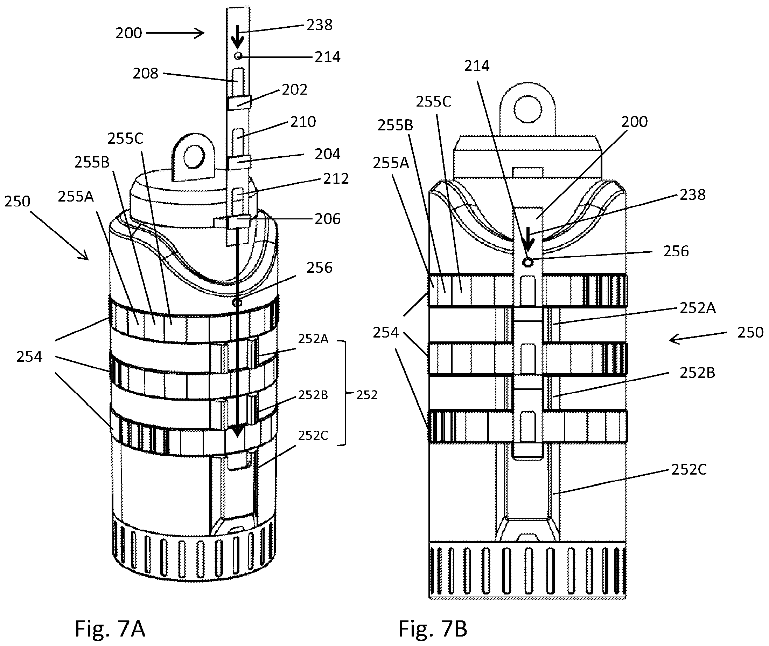

Referring now to Figures 7A and 7B, test strip 200 is shown in use with a comparator 250. The comparator is similar to that shown in Figure 1. However, the test strip holder 252 is shown with three separate portions 252A, 252B, 252C, which allows test strip 200 to be slid into the test strip holder, so that it overlies the moveable portions 254 of the comparator. Once the test strip 200 is slid into test strip holder 252, the aperture 214 engages with projection 256 to hold the test strip in place, as shown in Figure 7B. It can also be seen that, in this position, the transparent regions 208, 210, 212 overlie their respective moveable portions 254. The movable portions, on which the comparative results are located, and the results indicated on the testing regions 202, 204, 206 of the test strip 200 are now located adjacent to each other (i.e. the comparative results on the movable portion can be seen through the transparent regions), to simplify their comparison.

Whilst test strip holder 252 is shown having three separate portions 252A, 252B, 252C fewer, or more, separate portions may form the test strip holder. For example, only lower portion 252C may be used, or the lower portion may be used with projection 256, or only projection 256 may be used.

Whilst not shown, a non-windowed test strip, such as that shown in Figure 6D, may also be used with the comparator 250 shown in Figure 7. In such an embodiment, it may be preferable that either the indicator regions on the test strip or the movable collars 254 are adapted so that when the test strip is positioned in the test strip holder 252, the indicator regions and movable collars are substantially aligned, so that the indicated result overlies the potential colourimetric results shown on the movable collars. It may be preferable for each potential indicative colourimetric result, shown as bands 255A, 255B, etc, to be wider than the test strip. In such an embodiment, this may allow a simpler comparison of the indicated result positioned on either side thereof. As this would require a larger collar, it may be preferable that the variables be limited to, for example, one or two different pool volumes rather than three or four.

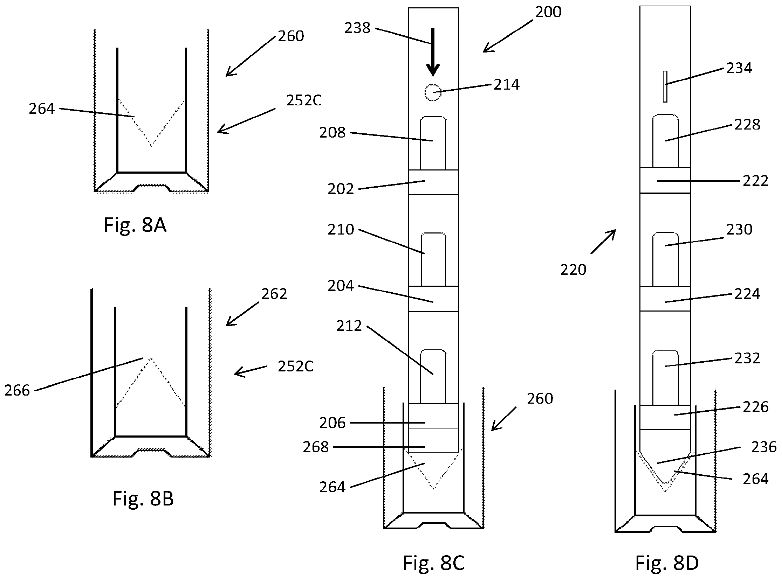

Figures 8A and 8B show enlarged schematic views of two embodiments 260, 262, respectively, of a lower portion 252C of a test strip holder 252, such as those shown in Figure 7. It can be seen that lower test strip holder portion 260 (Figure 8A) is suitable for use with a test strip 220, such as the one shown in Figure 6B, and lower test strip holder portion 262 (Figure 8B) is suitable for use with a test strip 260, such as the one shown in Figure 6D. Lower test strip holder portion 260 is shown having a recess 264 which is shaped to correspond to the tapered end 236 of test strip 220. The recess 264 may be integrally formed with the lower test strip holder portion 260, or may be in the form of a ledge attached to the lower test strip holder portion 260, or to the comparator 250 to which the test strip holder 252 is attached. Similarly, lower test strip holder portion 262 is shown having a rebate 266 that is shaped to correspond to the cutaway portion 240 of test strip 260.

Figures 8C and 8D show two different test strips 200, 220, respectively, being inserted into lower test strip holder portion 260. Test strips 200, 220 are similar to those depicted in Figures 6C and 6B, respectively, in that they each have three testing regions 202, 204 and 206; 222, 224 and 226, respectively, and they each have three transparent regions 208, 210 and 212; 228, 230 and 232, respectively.

Referring firstly to Figure 8C, test strip 200 has a flat base 268 and an arrow 238 to indicate the direction the test strip 200 should be placed into test strip holder 252, and more specifically into lower test strip holder portion 260. However, as lower test strip holder portion 260 has a recess 264, the test strip 200 is not able to be inserted all the way into lower test strip holder portion 260. This causes misalignment of the transparent regions 208, 210, 212 with movable collars 254. It also causes a misalignment of aperture 214 with projection 256 (on the comparator), thus preventing the test strip 200 from being held in the correct position. This indicates to the user that either the incorrect comparator or test strip is being used (as the arrow 238 was pointing in the correct direction for use).

Figure 8D again shows a lower test strip holder portion 260 having a recess 264. In this instance, test strip 220 has a correspondingly shaped tapered end 236. When the test strip 220 is inserted into the test strip holder 252, in the direction indicated by tapered end 236, the test strip is able to be fully inserted into lower test strip holder portion 260. This allows a proper alignment of the transparent regions 228, 230, 232 with movable collars 254 to allow comparison of the results on testing regions 222, 224, 226 with the comparative results on the movable collars 254 (i.e. by viewing the comparative results on the movable collars through the transparent regions). Further, rectangular aperture 234 is aligned with a corresponding projection on the comparator to hold the test strip 220 in the correct position.

Non-limiting Examples of the various comparators and dosage systems, in use, will now be described to illustrate how the comparator and dosage system may be applied, for example, to improve the water quality of a domestic swimming pool. It should, however, be appreciated that the comparator and dosage system can be used to improve the water quality of other bodies of water such as spas, ponds, aquariums, hot tubs, etc. While in the following examples test strips are used to measure various parameters indicative of water quality, other devices may also be used, such as electronic devices (hand-held or otherwise), sampling and specimen bottles, chemical test kits, etc.

In a general sense, the test strip(s) was placed in the water to be tested. The test strip was either directly dipped into the water in the swimming pool, or a small sample of the pool water was collected for testing and the test strip was dipped therein. The test strip was then removed from the water, and a predetermined length of time (e.g. as indicated by a supplied timing device) was allowed to pass before comparison of the test strip with the comparator.

### Example 1

A comparator, in the form of a test strip storage bottle was used, and a single test strip for testing pH was removed from the test strip storage bottle. The bottle had a movable collar at an outer portion thereof, the collar having two windows therein. The first window revealed a range of colour standards against which the test strip was to be compared, and the second window revealed the dosage of a water treating agent for treating the water.

The test strip was dipped into and swirled under the water for 10 seconds. Once the test strip was removed from the water, the test strip was held for 30 seconds, waiting for the colourimetric reaction (if any) to take place. The resultant colouring of the test strip was compared against various of the colour standards, each of which corresponded to various pH values, by moving the collar to reveal each of the various colour standards in the first window on the collar. As the collar was moved, the second window on the collar also moved, revealing the quantitative dosage of water treating agent, where appropriate corrective action was required to improve the quality of the water. The quantitative dosage indicated by the comparator was for a pool of 40,000L, as was being sampled.

Once the test strip had been compared to the comparator and matched to the closest colour result shown thereon, the quantitative indicated dosage was read from the second window when the water treating agent was to be added into the pool. In this example, the quantitative indicated dosage was four sachets of sodium bicarbonate.

The water treating agent, in this example sodium bicarbonate, was provided in sachet form. Thus, four sachets of sodium carbonate were added to the pool water, and were observed to improve the quality thereof. As the quantitative dosage was isolated when the test strip was compared to the comparator, it allowed the water treating agent to be added at leisure, without the concern of the test strip having overreacted.

### Example 2

A comparator in the form of a test strip bottle was used, and a single test strip capable of testing four parameters, such as chlorine, alkalinity, pH and total hardness, was removed from the test strip bottle located in the holder. The comparator was for a fixed volume of water (in this case 20,000L), such as a comparator shown in Figure 2. The bottle had four movable collars at an outer portion thereof, each collar having two windows therein. The first window of each collar revealed colour standards against which the test strip were compared, and the second window revealed the dosage of a water treating agent for treating the water.

The test strip was dipped into and swirled under the water for 20 seconds. Once the test strip was removed from the water, it was placed into a test strip holder on the side of the bottle holder to allow simple comparison of the measured result with the colour standards. The resultant colourings on the test strip in each testing region were compared to various colour standards, which corresponded to the various respective parameters: chlorine, alkalinity, pH and total hardness values, by rotating each of the collars to reveal the various colour standards in the first window on the collars. This was repeated for each test region, before any of the water treating agents were dosed into the pool, as it was imperative to ensure the test regions of the test strip were compared with the comparator before overreaction of the test region occurred.

As the collars were moved, the second window on the collars also moved, revealing a quantitative dosage of water treating agent, where appropriate corrective action was required to improve the quality of the water. Where no corrective action was required, the dosage window indicated "OK". Whilst the comparator indicated quantitative dosages for pools of 20,000L (the volume of water being sampled), other comparators were available to account for different volumes of water being sampled, water of different temperatures, summer or winter conditions, etc.

In this example, the total hardness result was within an acceptable range, so no corrective action was required (i.e. the quantitative dosage was indicated as "OK", as no dosing was required). However, as indicated by the comparator, 250 grams of sodium carbonate was required to adjust the pH, one scoop of sodium bicarbonate was required to adjust the alkalinity and two 1kg bottles of liquid chlorine were required to adjust the chlorine levels. These dosages of each water treating agent were added to the pool water, and were observed to improve the quality thereof.

### Example 3

A comparator in the form of a test strip storage bottle was used, and a single test strip able to test four different parameters, such as chlorine, alkalinity, pH and total hardness, was removed from the test strip storage bottle. Adjacent to each testing region on the test strip was a transparent region in the form of a cut-out window.

The comparator was for a fixed volume of water (in this case 20,000L), similar to the comparator shown in Figure 2. The bottle had four movable collars at an outer portion thereof, each collar having a window therein on an opposite side of the collar to the colour standards, and against which the test strip was to be compared. The window revealed the required dosage of a water treating agent for treating the water. The comparator also had a test strip holder that was configured such that, when holding the test strip, it overlied the moveable collars, whereby the transparent regions of the test strip were positioned over their respective movable collar.

The test strip was dipped into and swirled under the water for 20 seconds. Once the test strip was removed from the water, it was placed into the test strip holder on the side of the bottle holder to allow simple comparison of the measured result with each of the respective colour standards. The resultant colourings on the test strip in each testing region were compared to various colour standards, which corresponded to the various respective parameters: chlorine, alkalinity, pH and total hardness values. Each of the collars was rotated to reveal the various colour standards through the transparent region on the test strip, until the colour standard with the closest match to the colour of the testing region, was viewed through the transparent region. This was repeated for each testing region, and before any of the water treating agents were dosed into the pool, as it was imperative to ensure the test regions of the test strip were compared with the comparator before overreaction of the test region occurred.

As the collars were moved, the window on the opposite side of the collars also moved, revealing a quantitative dosage of water treating agent, if appropriate corrective action was required to improve the quality of the water. Where no corrective action was required, the dosage window indicated "OK". Whilst the comparator indicated quantitative dosages for pools of 20,000L (the volume of water being sampled), other comparators were available to account for different volumes of water being sampled, water of different temperatures, summer or winter conditions, etc.

In this example, the total hardness result was within an acceptable range, so no corrective action was required (i.e. the quantitative dosage was indicated as "OK", as no dosing was required). However, as indicated by the comparator, 250 grams of sodium carbonate was required to adjust the pH, one scoop of sodium bicarbonate was required to adjust the alkalinity and two 1kg bottles of liquid chlorine were required to adjust the chlorine levels. These dosages of each water treating agent were added to the pool water, and were observed to improve the quality thereof.

### Example 4

A single test strip for testing pH was removed from a test strip holder. A comparator, in the form of an adhesive label having two movable sleeves on a sodium bisulphate bottle, and indicating a quantitative dosage in the form of 'cap-fulls', was used for comparison with the test strip. An inner movable sleeve was configured to be rotated to alter the dosage indicated, to account for different volumes of water to be treated. The outer movable sleeve was configured to be rotated to indicate various colour standards, by aligning an indicator line against the resulting colour. When the colour standard that was the closest match to the result on the test strip was aligned with the indicator line, a second indicator line revealed the quantitative dosage of water treating agent required to improve the quality of the water. The comparator also had a small count-down timer provided thereon, which indicated the optimal time for comparing the result on the test strip with the comparator.

The test strip was dipped into and swirled under the water for 15 seconds. As soon as the test strip was removed from the water, the timer was pressed, resulting in the timer counting down from one minute. Once one minute had elapsed, an alarm indicated that it was time to compare the test strip with the comparator. The resultant colouring on the test strip was compared to various colour standards on the comparator, which corresponded to various pH values. As a pool of 30,000L was being sampled, the movable sleeve was rotated until the '30,000L' indicator line was aligned with a volume indicator on the adhesive label.

Once the test strip had been compared to the comparator and matched to the closest colour result shown thereon, the quantitative indicated dosage was read from the second window. In this example, the quantitative indicated dosage was 2 caps of granulated/powdered sodium bisulphate, used to lower the pH. The sodium bisulphate was poured into the cap to the 'fill line', dispensed, and repeated for the second 'cap full', to improve the pH level (by lowering it) of the pool water.

### Example 5

A pool having a volume of 80,000L was being tested, so a comparator for determining the dosage of water treating agent for a water volume of 80,000L was selected. The comparator had five distinct regions, the first being a compartment for storing unused test strips. The other four regions were comparative regions, configured to indicate the dosage of respective water treating agents required to treat the pool. Each comparative region had two windows, one window displayed indicative/comparative results (i.e. a given colour shading) and the other window displayed the dosage required for each result. Within each comparative region was a roller, adapted to display indicative/comparative results within the first window and the dosage of a water treating agent in the second window, the dosage being dependent upon the result obtained. Each roller was rotated via a respective knob. Each roller was also slidable in and out of each comparative region to alter the indicated dosage dependent on the frequency of use of the pool.

A single test strip capable of testing four parameters, such as pH, alkalinity, free chlorine and cyanuric acid, was removed from the test strip compartment. The test strip was dipped into and swirled under the water for 5 seconds. Once the test strip was removed from the water it was held for 20 seconds to allow the colourimetric response to take place. The resultant colourings of each section of the test strip were compared to their respective colour standards displayed in the first windows of each of the respective comparative regions, which corresponded to pH, alkalinity, free chlorine and cyanuric acid values.

Each testing portion of the test strip was compared to the corresponding colour standards for each comparative region of the comparator and matched to the closest colour result shown thereon by turning the knob on each respective roller. As the roller was rotated, the dosage indicated in the second window was also altered according to the result obtained. Further, each of the rollers was slid out of the comparative region to alter the indicated dosage due to the frequency of use. In this example, the roller was slid out of the comparative region until the marking indicating 'weekly usage' was visible on the roller. In this example, the pH result was within an acceptable range, so no corrective action was required (i.e. the quantitative dosage was indicated as '0' (zero); no dosing required). However, as indicated by the comparator, 1Kg of sodium bicarbonate was required to adjust the alkalinity, 1.5L of liquid chlorine was required to adjust the chlorine levels and 800mL of sodium hypochlorite were required to adjust the cyanuric acid levels. These dosages of each water treating agent were added to the pool water, and were observed to improve the quality thereof.

### Example 6

The comparator used had four distinct regions, each region being a comparative region configured to indicate the dosage of respective water treating agents required to treat the pool water. Each comparative region had two windows, one window displayed indicative/comparative results and the other window displayed the dosage required for each result. Within each comparative region was a roller, adapted to display indicative/comparative results within the first window and the dosage of a water treating agent in the second window, the dosage being dependent upon the result obtained. The dosage was further dependent on the volume of pool water to be treated. In this regard, another roller was provided that interacted with the indicated dosage via a gearing system to alter the dosage dependent on the volume of water. Each roller was rotated via a respective knob.

A single test strip capable of testing four parameters, such as pH, alkalinity, free chlorine and cyanuric acid, was placed in a test strip holder on the comparator. The test strip holder was arranged so that the test strip spanned the four comparative regions. The entire comparator, including the test strip, was dipped into and swirled under the water for 25 seconds. Once the comparator was removed from the water, the resultant colourings of each section of the test strip were compared to their respective colour standards, which corresponded to pH, alkalinity, free chlorine and cyanuric acid values. Each portion of the test strip was compared to the corresponding portion on the comparator and matched to the closest colour result shown thereon. To do so, the knob for each roller was turned until the colour on the roller matched the colour on the test strip. The knob for the volume was also turned, to change the dosage for the volume of the pool, in this case to 50,000L. In this example, the pH result was within an acceptable range, so no corrective action was required (i.e. the quantitative dosage was indicated as "OK"; no dosing required). However, as indicated by the comparator, one bucket of sodium bicarbonate was required to adjust the alkalinity, five scoops of liquid chlorine were required to adjust the chlorine levels and 10 pellets of sodium hypochlorite were required to adjust the cyanuric acid levels. These dosages of each water treating agent were added to the pool water, and were observed to improve the quality thereof.

### Example 7

A single test strip capable of testing three parameters, such as alkalinity, bromine and pH was removed from a test strip holder bottle. The test strip was dipped into and swirled under the water for 15 seconds. Once the test strip was removed from the water, the test strip was held for 30 seconds to allow the colourimetric response to take place. The resultant colouring of the test strip was compared to various colour standards on a comparator, which corresponded to various alkalinity, bromine and pH values. The comparator had four superimposed plastic discs, held together by a central axial plug, and was used for water volumes of 40,000L, the volume of the pool being sampled. The bottom disc of the comparator had an array of indicative/comparative results and the required quantitative dosages for rectifying pH values printed thereon. The third (i.e. from the top) disc had two windows therein, used to reveal the respective indicative/comparative results and quantitative dosages printed on the bottom disc. The third disc also had an array of indicative/comparative results and the required quantitative dosages for rectifying bromine levels printed thereon. The second (i.e. from the top) disc had two windows located therein, used to reveal the respective indicative/comparative results and the required dosages printed on the third disc. The second sheet also had an array of indicative/comparative results and required quantitative dosages for rectifying alkalinity levels printed thereon. The first (i.e. the top) disc had two windows located therein, used to reveal the respective indicative/comparative alkalinity results and quantitative dosages printed on the second disc.

The test strip result for pH was first compared with the indicative/comparative results for pH through the window on the third disc. In this regard, the third disc was rotated until the colour in the window that was the closest match to the colour on the test strip was obtained. The test strip result for bromine was then compared with the indicative/comparative results for bromine through the window on the second disc. In this regard, the second disc was rotated until the colour in the window that was the closest match to the colour on the test strip was obtained. Then, the test strip result for alkalinity was compared with the indicative/comparative results for alkalinity through the window on the first disc. In this regard, the first disc was rotated until the colour in the window that was the closest match to the colour on the test strip was obtained.

Once each of the results on the test strip had been compared with the comparator, the quantitative dosages required to rectify each parameter was read from the second window on each disc (i.e. the dosage from rectifying pH was read from the second window in disc three, the dosage for rectifying bromine was read from the second window in disc two, and the dosage for rectifying alkalinity was read from the second window in the first disc).

The comparator indicated that bromine levels were "OK", so no agent was required to alter the bromine levels. The comparator indicated that 750 grams of each of sodium bicarbonate and sodium carbonate were required to alter the alkalinity and pH, respectively. These dosages of each water treating agent were added to the pool water, and were observed to improve the quality thereof.

### Example 8

A comparator, either as a stand-alone test apparatus or in the form of a test strip storage bottle, were employed. A single test strip having four regions for testing four different parameters, such as chlorine, alkalinity, pH and calcium hardness, was removed from the test strip storage bottle. Adjacent to each testing region on the test strip was a transparent region, in the form of a see-through plastic material.

The comparator was for a fixed volume of water, in this case 30,000L. The comparator had four moveable collars at an outer portion thereof, with each collar having a window on an opposite side of the collar to the colour standards against which the test strip was to be compared. As the collars were moved, the window moved, revealing the required dosage of a water treating agent for treating the water, which was dependent on the alignment of the colour standard with the test strip. The comparator had a test strip holder which, when the test strip was correctly positioned therein, allowed each of the transparent regions of the test strip to overlie their respective moveable collar. The test strip also had a tapered end, which corresponded to a similarly shaped taper defined in a rebate of the test strip holder. With a first attempt, a test strip with a flat base was erroneously placed into the test strip holder, however, the transparent regions did not overlie the moveable collars and so it was readily determined by the user that the incorrect test strip had been placed into the test strip holder of the comparator.

A second test strip, which had a corresponding tapered end, was then correctly placed into the test strip holder and the transparent regions were correctly positioned to overlie the moveable collars.

In this example the entire test strip and comparator (e.g. when formed as a test apparatus) were dipped into and swirled under the water for fifteen seconds. Once the comparator, including the test strip was removed from the water, thirty seconds were allowed to lapse, to allow the colourimetric response of the test strip testing regions to take place. After thirty seconds, the resultant colourings on the test strip in each testing region were compared to the various colour standards on each collar. In this regard, each of the collars were rotated to reveal the various colour standards through the transparent region on the test strip, until the colour standard with the closest match to the colour of the testing region was viewed through the transparent region of the test strip.

As the collars were moved, the window of the opposite side of the collars also moved, and revealed a quantitative dosage of water treating agent, when appropriate corrective action was required to improve the quality of the water. When no corrective action was required, the dosage indicated "OK". Whilst the comparator indicated quantitative dosages for pools of 30,000 litres (the volume of the water being sampled, other comparators were available to account for different volumes of water being sampled, water of different temperatures, some more winter conditions, etc.

In this example, the calcium hardness result was outside its acceptable range, so corrective action was required to decrease the calcium hardness in the water. The comparator indicated that a 1L bottle of hydroxyethylidene diphosphonic acid complex was required. 250 grams of sodium bisulphate was required to adjust (lower) the pH, and three 1kg bottles of liquid chlorine were required to adjust (increase) the chlorine levels, as indicated by the comparator. 500mL of muriatic acid was also required to adjust (lower) the alkalinity. These dosages of each water treating agent were added to the pool water, and were observed to improve the quality thereof.

In the claims which follow, and in the preceding description, except where the context requires otherwise due to express language or necessary implication, the word "comprise" and variations such as "comprises" or "comprising" are used in an inclusive sense, i.e. to specify the presence of the stated features but not to preclude the presence or addition of further features in various embodiments of the comparator, system and method as disclosed herein.

## Claims

1. A comparator (10, 50, 70, 120, 160, 250) for use in improving the water quality of a body of water, the comparator being adapted for comparing one or more colourimetric results, the or each result corresponding to one or more measured parameter(s) indicative of water quality, the comparator being further adapted to indicate a quantitative dosage of water treating agent required to improve the quality of the water in response to one or more of the measured parameter(s),
**characterized in that** the comparator (10, 50, 70, 120, 160, 250) comprises an array of potential colourimetric results for each parameter measured and at least one movable portion which, when moved to the potential colourimetric result that corresponds to the colourimetric result of the parameter measured, indicates the quantitative dosage.

2. A comparator as claimed in claim 1 wherein the movable portion is a sleeve or collar.

3. A comparator as claimed in claim 1 or 2 further comprising at least one second moveable portion, that alters the indicated quantitative dosage to account for one or more factors which affect the quantitative dosage, wherein the one or more factors include: the volume of the body of water; water temperature; frequency of use; number of users; or seasonal factors.

4. A comparator as claimed in claim 3 wherein the second movable portion is a sleeve or collar.

5. A system for improving the water quality of a body of water in which a parameter indicative of the water quality is measured, the system comprising a comparator (10, 50, 70, 120, 160, 250) as claimed in any one of the preceding claims, and one or more test strips (26, 106, 200, 220, 260) for measuring the parameter indicative of the water quality.

6. A system as claimed in claim 5 wherein the or each test strip (26, 106, 200, 220, 260) is able to test for one or more of the parameters indicative of water quality at a respective indicator region.

7. A system as claimed in claim 5 or 6 comprising a vessel (12, 52), the comparator being located on said vessel.

8. A system as claimed in claim 7 comprising a test strip holder (62, 252) located on a visible portion of the vessel.

9. A system as claimed in claim 8 wherein the test strip holder(62, 252) is positioned such that the test strip, when positioned therein, overlies the indicated results/movable portion.

10. A system as claimed in claim 8 wherein the test strip holder (62, 252) is positioned such that the or each movable portion overlies the test strip, when the test strip is positioned at or in the holder.

11. A system as claimed in any one of claims 7 to 10 wherein the vessel (12, 52) is a holder for unused test strips for measuring the one or more parameters.

12. A system as claimed in any one of claims 7 to 11 wherein the vessel (12, 52) contains one or more of the water treating agents.

13. A method of improving the water quality of a body of water comprising comparing a colourimetric result of a measured parameter, the parameter being indicative of water quality, with a comparator (10, 50, 70, 120, 160, 250) as claimed in any ones of claims 1 to 4 by moving a movable portion of the comparator to correspond to the colourimetric result of the parameter measured, thus indicating the quantitative dosage of a water treating agent required to improve the quality of the water.

## Patentansprüche

1. Komparator (10, 50, 70, 120, 160, 250) zur Verwendung beim Verbessern der Wasserqualität eines Gewässers, wobei der Komparator dafür geeignet ist, ein oder mehrere kolorimetrische Ergebnisse zu vergleichen, wobei das oder jedes Ergebnis einem oder mehreren gemessenen Parametern entspricht, die eine Wasserqualität anzeigen, wobei der Komparator weiter dazu geeignet ist, in Antwort auf einen oder mehrere der gemessenen Parameter eine mengenmäßige Dosierung von Wasserbehandlungsmittel anzuzeigen, die erforderlich ist, um die Qualität des Wassers zu verbessern, **dadurch gekennzeichnet, dass** der Komparator (10, 50, 70, 120, 160, 250) eine Reihe von möglichen kolorimetrischen Ergebnissen für jeden gemessenen Parameter und mindestens einen beweglichen Abschnitt umfasst, der, wenn er zu dem möglichen kolorimetrischen Ergebnis bewegt wird, das dem kolorimetrischen Ergebnis des gemessenen Parameters entspricht, die mengenmäßige Dosierung anzeigt.

2. Komparator nach Anspruch 1, wobei der bewegliche Abschnitt eine Hülse oder Manschette ist.

3. Komparator nach Anspruch 1 oder 2, weiter mindestens einen zweiten beweglichen Abschnitt umfassend, der die angezeigte mengenmäßige Dosierung verändert, um einen oder mehrere Faktoren zu berücksichtigen, die sich auf die mengenmäßige Dosierung auswirken, wobei der eine oder die mehreren Faktoren einschließen: das Volumen des Gewässers; die Wassertemperatur; die Nutzungshäufigkeit; die Nutzeranzahl; oder saisonale Faktoren.

4. Komparator nach Anspruch 3, wobei der zweite bewegliche Abschnitt eine Hülse oder Manschette ist.

5. System zum Verbessern der Wasserqualität eines Gewässers, wobei ein Parameter gemessen wird, der die Wasserqualität anzeigt, wobei das System einen Komparator (10, 50, 70, 120, 160, 250) nach einem der vorstehenden Ansprüche und einen oder mehrere Teststreifen (26, 106, 200, 220, 260) zum Messen des die Wasserqualität anzeigenden Parameters umfasst.

6. System nach Anspruch 5, wobei der oder jeder Teststreifen (26, 106, 200, 220, 260) in der Lage ist, in einem jeweiligen Anzeigebereich auf einen oder mehrere der die Wasserqualität anzeigenden Parameter zu testen.

7. System nach Anspruch 5 oder 6, das einen Behälter (12, 52) umfasst, wobei sich der Komparator am Behälter befindet.

8. System nach Anspruch 7, das einen Teststreifenhalter (62, 252) umfasst, der sich an einem sichtbaren Abschnitt des Behälters befindet.

9. System nach Anspruch 8, wobei der Teststreifenhalter (62, 252) derart positioniert ist, dass der Teststreifen, wenn er in demselben positioniert ist, die angezeigten Ergebnisse/den beweglichen Abschnitt überlagert.

10. System nach Anspruch 8, wobei der Teststreifenhalter (62, 252) derart positioniert ist, dass der oder jeder bewegliche Abschnitt den Teststreifen überlagert, wenn der Teststreifen am oder im Halter positioniert ist.

11. System nach einem der Ansprüche 7 bis 10, wobei der Behälter (12, 52) ein Halter für ungebrauchte Teststreifen zum Messen des einen oder der mehreren Parameter ist.

12. System nach einem der Ansprüche 7 bis 11, wobei der Behälter (12, 52) eines oder mehrere der Wasserbehandlungsmittel enthält.

13. Verfahren zum Verbessern der Wasserqualität eines Gewässers, das das Vergleichen eines kolorimetrischen Ergebnisses eines gemessenen Parameters, wobei der Parameter die Wasserqualität anzeigt, mit einem Komparator (10, 50, 70, 120, 160, 250) nach einem der Ansprüche 1 bis 4 durch Bewegen eines beweglichen Abschnitts des Komparators so umfasst, dass derselbe dem kolorimetrischen Ergebnis des gemessenen Parameters entspricht, wodurch die mengenmäßige Dosierung eines Wasserbehandlungsmittels angezeigt wird, die erforderlich ist, um die Qualität des Wassers zu verbessern.

## Revendications

1. Comparateur (10, 50, 70, 120, 160, 250) pour son utilisation dans l'amélioration de la qualité de l'eau d'une masse d'eau, le comparateur étant adapté pour comparer un ou plusieurs résultats colorimétriques, le ou chaque résultat correspondant à un ou plusieurs paramètres mesurés indicateur de la qualité de l'eau, le comparateur étant adapté en outre pour indiquer un dosage quantitatif d'un agent de traitement d'eau nécessaire pour améliorer la qualité de l'eau en réponse à un ou plusieurs du ou des paramètres mesurés,
**caractérisé en ce que** le comparateur (10, 50, 70, 120, 160, 250) comprend une série de résultats colorimétriques potentiels pour chaque paramètre mesuré et au moins une partie mobile qui, quand elle est déplacée vers le résultat colorimétrique potentiel qui correspond au résultat colorimétrique du paramètre mesuré, indique le dosage quantitatif.

2. Comparateur selon la revendication 1 dans lequel la partie mobile est un manchon ou un collier.

3. Comparateur selon la revendication 1 ou 2 comprenant en outre au moins une deuxième partie mobile, qui modifie le dosage quantitatif indiqué pour prendre en compte un ou plusieurs facteurs qui affectent le dosage quantitatif, dans lequel les un ou plusieurs facteurs incluent : le volume de la masse d'eau ; la température de l'eau ; la fréquence d'utilisation ; le nombre d'utilisateurs ; ou des facteurs saisonniers.

4. Comparateur selon la revendication 3 dans lequel la deuxième partie mobile est un manchon ou un collier.

5. Système pour améliorer la qualité de l'eau d'une masse d'eau dans lequel un paramètre indicateur de la qualité de l'eau est mesuré, le système comprenant un comparateur (10, 50, 70, 120, 160, 250) selon l'une quelconque des revendications précédentes, et une ou plusieurs bandelettes réactives (26, 106, 200, 220, 260) pour mesurer le paramètre indiquant la qualité de l'eau.

6. Système selon la revendication 5 dans lequel la ou chaque bandelette réactive (26, 106, 200, 220, 260) est capable de tester un ou plusieurs des paramètres indicateur de la qualité de l'eau au niveau d'une région indicatrice respective.

7. Système selon la revendication 5 ou 6 comprenant un récipient (12, 52), le comparateur étant situé sur ledit récipient.

8. Système selon la revendication 7 comprenant un support de bandelette réactive (62, 252) situé sur une partie visible du récipient.

9. Système selon la revendication 8 dans lequel le support de bandelette réactive (62, 252) est positionné de façon que la bandelette réactive, quand elle est positionnée à l'intérieur, recouvre les résultats indiqués/la partie mobile.

10. Système selon la revendication 8 dans lequel le support de bandelette réactive (62, 252) est positionné de façon que la ou chaque partie mobile recouvre la bandelette réactive, quand la bandelette réactive est positionnée au niveau du support ou dans celui-ci.

11. Système selon l'une quelconque des revendications 7 à 10 dans lequel le récipient (12, 52) est un support pour bandelettes réactives non utilisées pour mesurer les un ou plusieurs paramètres.

12. Système selon l'une quelconque des revendications 7 à 11 dans lequel le récipient (12, 52) contient un ou plusieurs des agents de traitement d'eau.

13. Procédé d'amélioration de la qualité de l'eau d'une masse d'eau comprenant la comparaison d'un résultat colorimétrique d'un paramètre mesuré, le paramètre indiquant la qualité de l'eau, avec un comparateur (10, 50, 70, 120, 160, 250) selon l'une quelconque des revendications 1 à 4 par le déplacement d'une partie mobile du comparateur pour correspondre au résultat colorimétrique du paramètre mesuré, pour ainsi indiquer le dosage quantitatif d'un agent de traitement d'eau nécessaire pour améliorer la qualité de l'eau.
